(19) Europäisches Patentamt
European Patent Office
Office européen des brevets

(11) Numéro de publication: **0 323 294 A1**

# (12) DEMANDE DE BREVET EUROPEEN

(21) Numéro de dépôt: **88403103.0**

(22) Date de dépôt: **07.12.88**

(51) Int. Cl.⁴: **A 61 K 7/48**
A 61 K 7/00

(30) Priorité: **07.12.87 FR 8716969**

(43) Date de publication de la demande:
**05.07.89 Bulletin 89/27**

(84) Etats contractants désignés:
**AT BE CH DE ES FR GB GR IT LI LU NL SE**

(71) Demandeur: **PIERRE FABRE COSMETIQUE**
**125 rue de la Faisanderie**
**F-75116 Paris (FR)**

(72) Inventeur: **Trébosc, Marie-Thérèse**
**19, rue Baron Cachin**
**F-81100 Castres (FR)**

**Cousse, Henri**
**La Foun de los Noblos Chemin de Lastinos**
**F-81100 Castres (FR)**

**Mouzin, Gilbert**
**11, rue des Pénitents blancs**
**F-31000 Toulouse (FR)**

(74) Mandataire: **Ahner, Francis et al**
**CABINET REGIMBEAU 26, avenue Kléber**
**F-75116 Paris (FR)**

Le titre de l'invention a été modifié (Directives relatives à l'examen pratiqué à l'OEB, A-III, 7.3)

(54) **Compositions topiques hétérogènes à action amincissante et/ou anti-cellulitique à base de microgranules d'un sel de l'acide caféine carboxylique, ainsi que leur préparation.**

(57) La présente invention concerne des compositions topiques hétérogènes à base de microgranules d'un sel de l'acide caféine carboxylique, utiles comme amincissant et/ou dans le traitement de la cellulite, ainsi que leur préparation.

La composition topique hétérogène, selon l'invention, utile comme amincissant et/ou dans le traitement de la cellulite, est caractérisée en ce qu'elle comporte à titre de principes actifs d'un sel de l'acide caféine carboxylique, lesdits principes actifs étant présents sous la forme de microgranules ou de microparticules en suspension au sein d'un gel hydroalcoolique.



Bundesdruckerei Berlin

**Description**

**COMPOSITIONS TOPIQUES HETEROGENES A BASE DE MICROGRANULES DE CAFEINE ET/OU DE SES DERIVES, UTILES COMME AMINCISSANT ET/OU DANS LE TRAITEMENT DE LA CELLULITE, AINSI QUE LEUR PREPARATION**

La présente invention, réalisée au Centre Dermatologique et Cosmétologique PIERRE FABRE, concerne des nouvelles formulations cosmétiques dans lesquelles le ou les principes actifs sont maintenus en suspension au sein d'un gel hydroalcoolique, sous la forme de microgranules ou de microparticules.

Les principes actifs sont principalement constitués par la caféine et ses dérivés insolubles, éventuellement en association avec la vitamine E et/ou ses dérivés.

Dans l'état de la technique antérieure, les préparations amincissantes étaient constituées par des gels translucides hydroalcooliques d'aspect homogène. Selon la présente invention, le principe actif est visualisé dans la composition amincissante, sous la forme de microgranules ou autres préparations galéniques permettant d'obtenir des produits peu solubles ou insolubles dans les excipients hydroalcooliques.

Les principes actifs utilisés dans le cadre de la présente invention, sont la caféine et ses dérivés de formule générale (I):

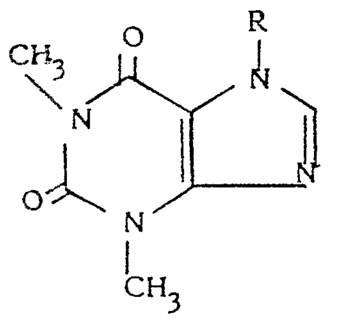

(I)

dans laquelle :
R représente un groupe ($C_1$-$C_4$ alcoyle)carboxylique et ses sels, plus particulièrement les sels de zinc, magnésium, aluminium et calcium.

La préparation des nouveaux principes actifs utilisés dans la présente invention est illustrée par les exemples suivants.

**Exemple 1**

Préparation de caféine carboxylate de zinc

On solubilise 238,2 g (1mole) d'acide caféine carboxylique dans un litre de soude (N) sous agitation. A la solution limpide orangée, on ajoute 143,7g (0,5 mole) de sulfate de zinc hydraté en solution dans 150 ml d'eau. On laisse sous forte agitation pendant une heure, il se forme un précipité blanc ; après filtration et séchage, on récupère quantitativement le produit de formule :

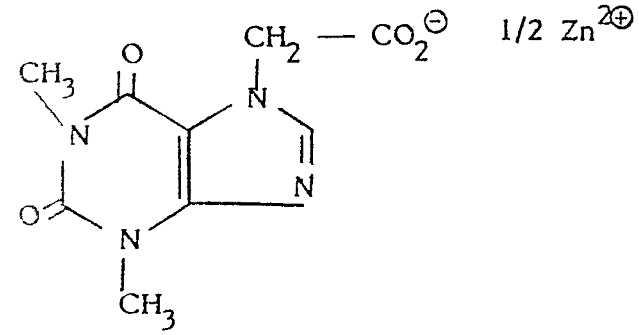

Formule brute : $C_{18}$ $H_{18}$ $N_8$ $O_8$ Zn    PM : 539,76
Caractéristiques physico-chimiques
Couleur : blanc    Point de fusion > 260°C

Analyse élémentaire (Lot FII 137)

| | C | H | N | Zn |
|---|---|---|---|---|
| Calculée | 40,05 | 3,36 | 20,76 | 12,11 |
| Trouvée | conforme avec 28,8% d'eau | | | |

Chromatographie en couche mince :Gel de silice 60 F 254 MERCK Réf.: 0,68
Solvant : chloroforme - méthanol 50/50
Révélation : UV
Solubilités
Eau : à 0,1%, Ethanol à 30° insoluble
huile d'arachide : insoluble.

D'une manière similaire à celle décrite dans l'exemple 1, les produits suivants ont été préparés.

**Exemple 2** : Caféine carboxylate de magnésium

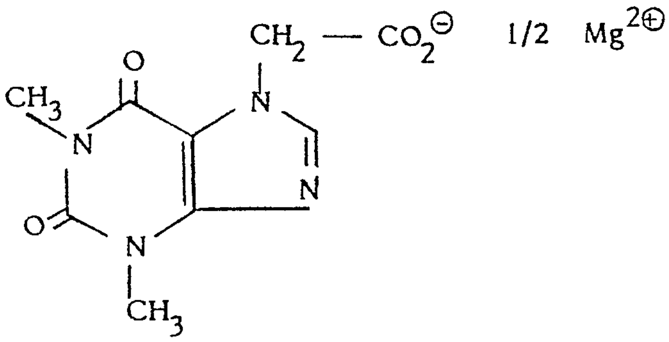

Formule brute : $C_{18} H_{18} N_8 O_8 Mg$ PM : 498,71
Caractéristiques physico-chimiques
Couleur : blanc Point de fusion > 260° C

Analyse élémentaire (Lot FII 135)

| | C | H | N | Mg |
|---|---|---|---|---|
| Calculée | 43,35 | 3,64 | 22,47 | 4,87 |
| Trouvée | conforme avec 34% d'eau | | | |

Chromatographie en couche mince :Gel de silice 60 F 254 MERCK Réf.: 0,68
Solvant : $CHCl_3$ - MeOH 50/50
Révélation : UV
Solubilités
Eau : 0,5% Ethanol 0,5%
huile d'arachide : insoluble.

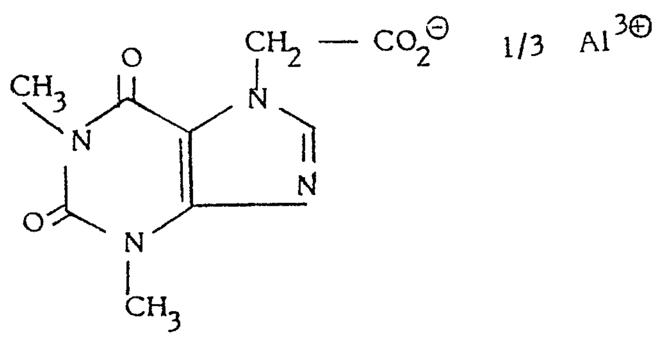

Formule brute : $C_{27}H_{27}N_{12}O_{12}Al$ PM 738,57
Caractéristiques physico-chimiques
Couleur : blanc Point de fusion > 260°C

Analyse élémentaire (Lot FII 150)

| | C | H | N | Al |
|---|---|---|---|---|
| Calculée | 43,12 | 3,62 | 22,35 | 5,38 |
| Trouvée | conforme avec 13.4% d'eau | | | |

Chromatographie en couche mince :Gel de silice 60 F 254 MERCK Réf.: 0,68
Solvant : $CHCl_3$ - MeOH 50/50
Révélation : UV
Solubilités
Eau : 3% ; Ethanol 30°C : 1%
huile d'arachide : insoluble.

**Exemple 4** : Caféine carboxylate de calcium

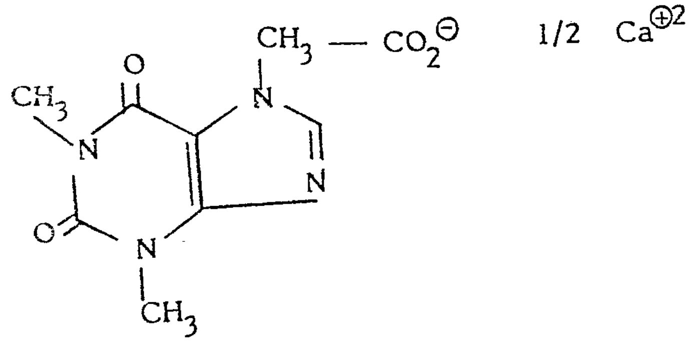

Formule brute : $C_{18}H_{18}N_8O_8Ca$ PM : 550,48
Caractéristiques physico-chimiques
Couleur : blanc Point de fusion > 300°C

Analyse élémentaire (Lot AXXI 121)

| | C | H | N | Ca |
|---|---|---|---|---|
| Calculée | 39,27 | 4,03 | 29,35 | 7,28 |
| Trouvée | conforme avec 7.3% d'eau | | | |

Chromatographie en couche mince :Gel de silice 60 F 254 MERCK Réf.: 0,68
Solvant $CHCl_3$ - MeOH 50/50
Révélation : UV
Solubilités
Eau : 2% ; Ethanol 30°C : 1%
huile d'arachide insoluble.

Selon la présente invention, les préparations galéniques hétérogènes à usage cosmétique contiennent comme principes actifs des dérivés de la caféine de formule générale 1, associés ou non à des dérivés de la vitamine E, lesdits principes actifs étant présents sous forme de microgranules, microparticules ou entités analogues insolubles ou très difficilement solubles dans le gel hydroalcoolique de base.

Ces principes actifs sont ainsi libérés de façon progressive et prolongée, lors de l'application topique.

Une telle libération prolongée est obtenue grâce à la préparation de particules hétérogènes comportant au moins deux phases solides.

La première phase comporte un support liant inerte thermoplastique et la seconde phase comporte des particules imprégnées de principes actifs et/ou des particules de principes actifs purs.

Le procédé de préparation de telles formulations comporte les étapes suivantes :

1. La première phase est micronisée jusqu'à l'obtention de lamelles de taille moyenne inférieure à 50 µm.

2. Les particules de charge imprégnées d'actifs et/ou les principes actifs purs sont micronisées jusqu'à l'obtention de particules de taille moyenne inférieure à 50 µm.

3. Les lamelles obtenues au cours de l'étape 1 sont mélangées avec les particules obtenues au cours de l'étape 2.

4. L'éthanol est ajouté comme agent mouillant, après séchage et ventilation, on récupère une poudre qui est calibrée par tamisage pour obtenir une taille de particule comprise entre 0,2 et 2 mm.

Les principes actifs purs ou les particules hétérogènes chargées d'actifs microgranulés selon la présente invention sont de préférence :

. des principes actifs à visée amincissante et/ou utiles dans le traitement de la cellulite, et principalement : la caféine, le caféine carboxylate de zinc, le caféine carboxylate de magnésium, le caféine carboxylate d'aluminium, le caféine carboxylate de calcium, éventuellement associé à

. des vitamines et plus particulièrement la vitamine E et/ou ses dérivés.

Les formulations comportent de 0,5 à 20% p/p de microgranules chargées d'actifs ou d'actifs purs dans une base appropriée comportant des excipients cosmétiques connus de l'homme de l'art : cette base est choisie de telle sorte que les particules y soient insolubles.

A titre d'exemples non limitatifs sont citées les formulations amincissantes de gel hydroalcoolique contenant de 1 à 10% de microgranules dont les compositions sont les suivantes :

Exemple 1

| | |
|---|---|
| Caféine | 50 à 70% |
| Amidon | 20 à 30% |
| Ethylcellulose | 5 à 20% |

Exemple 2

| | |
|---|---|
| Caféine carboxylate de zinc | 100% |

Exemple 3

| | |
|---|---|
| Caféine carboxylate de zinc | 60 à 70% |
| Vitamine E | 30 à 40% |

Exemple 4

| | |
|---|---|
| Caféine carboxylate de magnésium | 50 à 80% |
| Amidon | 20 à 50% |

Exemple 5

| | |
|---|---|
| Caféine | 50 à 70% |
| Vitamine E | 20 à 30% |
| Amidon | 5 à 20% |

Les formulations selon la présente invention sont bien tolérées, elles mettent en jeu des microparticules molles ne faisant intervenir aucun agent minéral susceptible d'être considéré comme corps étranger.

Selon la présente invention, les formulations préparées compte tenu de la présence de principes actifs, tels que caféine et ses dérivés, possèdent d'excellentes propriétés "lipolytiques" et se sont donc révélées très efficaces dans le traitement de la cellulite.

**Revendications**

1/ Composition topique hétérogène, utile comme amincissant et/ou dans le traitement de la cellulite, caractérisée en ce qu'elle comporte à titre de principes actifs des sels métalliques de l'acide caféine carboxylique, lesdits principes actifs étant présents sous la forme de microgranules ou de microparticules en suspension au sein d'un gel hydroalcoolique.

2/ Composition selon la revendication 1, caractérisée en ce que lesdites compositions contiennent en outre de la vitamine E et/ou de la caféine et/ou l'un de ses dérivés.

3/ Composition selon l'une des revendications 1 et 2, caractérisée en ce que les dérivés de l'acide caféine carboxylique répondent à la formule générale suivante :

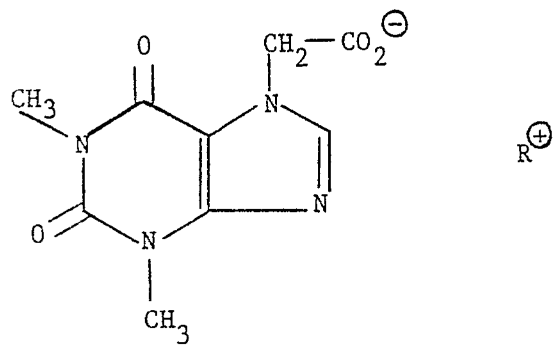

dans laquelle :
$R^{\oplus}$ représente un cation métallique cosmétologiquement acceptable.

4/ Composition selon la revendication 3, caractérisée en ce que le dérivé de la caféine est plus particulièrement le caféine carboxylate de zinc.

5/ Composition selon la revendication 3, caractérisée en ce que le dérivé de la caféine est le caféine carboxylate de magnésium.

6/ Composition selon la revendication 3, caractérisée en ce que le dérivé de la caféine est le caféine carboxylate d'aluminium.

7/Composition selon la revendication 3, caractérisée en ce que le dérivé de la caféine est le caféine carboxylate de calcium.

8/Procédé de préparation de microgranules contenant des actifs selon l'une des revendications 1 à 7, caractérisé en ce que la préparation des microgranules comprend les étapes suivantes :

a) La première phase comportant un support liant inerte est micronisée jusqu'à l'obtention de lamelles de taille moyenne inférieure à 50 µm.

b) Les particules de charge imprégnées d'actifs et/ou les principes actifs purs sont micronisées jusqu'à l'obtention de particules de taille moyenne inférieure à 50 µm.

c) Les lamelles obtenues au cours de l'étape a) sont mélangées avec les particules obtenues au cours de l'étape b).

d) L'éthanol est ajouté comme agent mouillant, après séchage, la poudre obtenue est tamisée pour former des microgranules comprises entre 0,2 et 2 mm de diamètre.

Office européen des brevets

# RAPPORT DE RECHERCHE EUROPEENNE

Numero de la demande

EP 88 40 3103

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 554 344 (P.F. COSMETIQUE) * Page 2, ligne 30; revendications 1-7 * --- | 1,3 | A 61 K 7/48<br>A 61 K 7/00 |
| Y | FR-A-2 490 492 (RIKER LABORATORIES) * En entier * --- | 1,3 | |
| Y | FR-A-2 563 104 (SHISEIDO) * Revendications * --- | 1,3 | |
| Y | US-A-3 084 104 (TUERCK et al.) * Revendication 1 * --- | 8 | |
| Y | DE-A-1 667 125 (KÖHLER) * En entier * --- | 8 | |
| Y | DE-A-1 492 045 (KONINKLIJKE PHARMACEUTISCHE FABRIEKEN V/H BROCADES-STHEEMAN & PHARMACIA) * Revendication 1 * --- | 8 | **DOMAINES TECHNIQUES RECHERCHES (Int. Cl.4)** |
| P,X | FR-A-2 611 497 (P.F. COSMETIQUE) * En entier * --- | 8 | A 61 K |
| A | CHEMICAL ABSTRACTS, vol. 99, no. 10, septembre 1983, page 358, no. 76745m, Columbus, Ohio, US; C. SIREGAR et al.: "Effect of viscous binding agent viscosity on the physical properties of theophylline-containing granules and on the rate of theophylline dissolution from the granules", & ACTA PHARM. INDONES. 1983, 8(1), 23-41 ----- | 8 | |

Le présent rapport a été établi pour toutes les revendications

| Lieu de la recherche | Date d'achèvement de la recherche | Examinateur |
|---|---|---|
| LA HAYE | 10-03-1989 | FISCHER J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant